# EUROPÄISCHE PATENTSCHRIFT

(11) **EP 0 712 848 B1**
(45) Veröffentlichungstag und Bekanntmachung des Hinweises auf die Patenterteilung: **27.09.2000**
(21) Anmeldenummer: 95117130.5
(22) Anmeldetag: 31.10.1995
(51) Int. Cl.: C07D 235/26

(54) **Verfahren zur Herstellung von 3-Hydroxy-N-benzimidazolon-5-yl-napthoesäure-2-amid in hoher für Azopigmente erforderlicher Reinheit**
Process of preparation of 3-hydroxy-N-benzimidazolon-5-yl-naphth-2-amides, in high purity for the preparation of azodyes
Procédé pour la préparation des 3-hydroxy-N-benzimidazolon-5-yl-napht-2-amides, en très grande pureté, nécessaire pour la préparation des colorants azoiques

(30) Priorität: 18.11.1994 DE 4441146
(43) Veröffentlichungstag der Anmeldung: 22.05.1996
(73) Patentinhaber: Clariant GmbH, 65929 Frankfurt am Main (DE)
(72) Erfinder: Meier, Michael, Dr., D-60487 Frankfurt (DE); Volk, Heinrich, Dr., D-61118 Bad Vilbel (DE); Neeb, Rudolf, Dr., D-63075 Offenbach (DE)

(56) Entgegenhaltungen:
- EP-A- 0 002 747
- DD-A- 265 894
- DYES AND PIGMENTS, Bd. 12, 1990, Seite 57-63 XP000570451 JAN KRASKA ET AL.: "Synthesis of Amides of 3-Hydroxy-2-naphthoic acid: Derivatives of Benzimidazolone and Benzoxazolone."
- REVUE ROUMAINE DE CHIMIE, Bd. 37, Nr. 6, Juni 1992, BUCAREST, Seiten 719-722, XP000196260 M. VATA ET AL.: "Unkonventionelle Synthese des 5-Amino-Benzimidazolon-(2)-naphtharylids"
- CHEMICAL ABSTRACTS, vol. 92, no. 9, 3.März 1980 Columbus, Ohio, US; abstract no. 76179k, Seite 647; Spalte 2; XP002017538 & CS-A-178 560 (M. CHLOST)
- CHEMICAL ABSTRACTS, vol. 112, no. 13, 26.März 1990 Columbus, Ohio, US; abstract no. 118816e, Seite 741; Spalte 2; XP002017539 & PL-A-144 734 (POLITECHNIKA LODZKA)

## Beschreibung

Die Erfindung betrifft ein Verfahren zur Herstellung von 3-Hydroxy-N-benzimidazolon-5-yl-naphthoesäure-2-amid in hoher für Azopigmente erforderlicher Reinheit durch Umsetzung von 3-Hydroxy-2-naphthoesäure in Toluol bzw. Xylol mit Thionylchlorid und anschließender Kondensation mit 5-Aminobenzimidazolon in N-Methylpyrrolidon in Anwesenheit von Natriumcarbonat.

3-Hydroxy-N-benzimidazolon-5-yl-naphthoesäure-2-amid ist ein wichtiges Zwischenprodukt für die Herstellung von hochechten Azopigmenten.

Es ist bekannt, daß man 3-Hydroxy-N-benzimidazolon-5-yl-naphthoesäure-2-amid durch Umsetzung von 3-Hydroxy-2-naphthoesäure und 5-Aminobenzimidazolon in N-Methylpyrrolidon mit Phosphortrichlorid bei Temperaturen von 105-150°C in 84 % Ausbeute in einer gelben Modifikation, die für Azopigmente tauglich ist, herstellen kann (DE 2758818). Ebenso ist bekannt, daß man 3-Hydroxy-N-benzimidazolon-5-yl-naphthoesäure-2-amid durch Reaktion von stark überschüssiger 3-Hydroxy-2-naphthoesäure und 5-Aminobenzimidazolon in Xylol mit Phosphortrichlorid in 84 % Ausbeute herstellen kann (DD 265894). Nachteilig an diesem Verfahren ist, daß die überschüssige 3-Hydroxy-2-naphthoesäure wieder zurückgewonnen werden muß, wodurch das Verfahren ökonomisch ungünstig wird. Bei beiden Verfahren entsteht ein phosphorhaltiges Abwasser.
In der CS 178560 wird die Umsetzung von einer Lösung von 3-Hydroxy-2-naphthoesäurechlorid in Toluol mit 5-Aminobenimidazolon in der 30 fachen Menge Wasser in Anwesenheit von Natriumacetat zu 3-Hydroxy-N-benzimidazolon-5-yl-naphthoesäure-2-amid in 86 % Ausbeute beschrieben. Nachteilig an diesem Verfahren sind die großen Reaktionsvolumina.

Die Umsetzung von stöchiometrischen Mengen 3-Hydroxy-2-naphthoesäurechlorid und 5-Aminobenzimidazolen in organischen Säuren in Gegenwart der Natrium- oder Kaliumsalze dieser Säuren (Polnische P 119895) ist bekannt. Eine Verbesserung dieser Herstellungsmethode besteht im Ersatz der organischen Säuren durch N,N'-Dimethylformamid bzw. N-Methylpyrrolidon (Polnische P 144734 und Dyes and Pigments 12, 57-63 (1990)). Das nach dieser Herstellmethode hergestellte 3-Hydroxy-N-benzimidazolon-5-yl-naphthoesäure-2-amid entspricht nicht den Anforderungen für Azopigmente, da das Produkt noch zuviel Ausgangsmaterial enthält (siehe Vergleichsbeispiel 1). Ein weiterer Nachteil dieses Verfahrens sind die langen Saugzeiten bei der Isolation des Produktes (siehe Vergleichsbeispiel 1.)

Bei den meisten obengenannten Verfahren entsteht 3-Hydroxy-N-benzimidazolon-5-yl-naphthoesäure-2-amid nicht in der erforderlichen hohen Reinheit für Azopigmente. Deshalb müssen diese Produkte für die Herstellung hochechter Azopigmente, wie in der DE 2758818 beschrieben, über das Alkalisalz gereinigt werden.

Es bestand daher ein Bedürfnis nach einem wirtschaftlichen und technisch leicht durchführbaren Verfahren zur Herstellung von 3-Hydroxy-N-benzimidazolon-5-yl-napthoesäure-2-amid in einer sehr hohen Reinheit für die Herstellung von hochechten Azopigmenten.

Diese Aufgabe wird gelöst durch ein Verfahren zur Herstellung von 3-Hydroxy-N-benzimidazolon-5-yl-naphthoesäure-2-amid, ausgehend von 5-Aminobenzimidazolon und 3-Hydroxy-2-naphthoesäurechlorid, dadurch gekennzeichnet, daß man 5-Aminobenzimidazolon und Natriumcarbonat in N-Methylpyrrolidon vorlegt und eine Lösung von 3-Hydroxy-2-naphthoesäurechlorid in Xylol oder Toluol innerhalb von mindestens 2 Stunden zudosiert, filtriert, mit Xylol wäscht, den Filterkuchen in Wasser, das mit Natriumcarbonat versetzt ist, einträgt und wasserdampfdestilliert, den Rückstand absaugt, wäscht und trocknet.

3-Hydroxy-2-naphthoesäurechlorid wird nach an sich bekannter Weise aus 1 mol 3-Hydroxy-2-naphthoesäure in ca. 5 Volumenteilen Xylol bzw. Toluol pro Teil 3-Hydroxy-2-naphthoesäure durch Umsetzung mit 1,05 Thionylchlorid unter Zusatz eines Katalysators, wie z. B. N,N'-Dimethylformamid, Pyridin, Triethylamin, bei 47-50°C hergestellt. Die so hergestellte Lösung wird weiter mit 5-Aminobenzimidazolon umgesetzt.

Üblicherweise verfährt man bei der Herstellung von 3-Hydroxy-N-benzimidazolon-5-yl-naphthoesäure-2-amid so, daß man 5-Aminobenzimidazolon und Natriumcarbonat in N-Methylpyrrolidon vorlegt und eine Lösung von 3-Hydroxy-2-naphthoesäurechlorid in Xylol bzw. Toluol in 4 bis 10 h zudosiert. Anschließend wird filtriert, mit Xylol bzw. Toluol gewaschen und der Filterkuchen in Wasser eingetragen, mit Natriumcarbonat versetzt und destilliert. Es wird erneut abgesaugt, der Filterkuchen vorzugsweise mit Wasser neutral gewaschen und getrocknet.

5-Aminobenzimidazolon wird in einer Menge von 0,9 bis 1 mol, insbesondere 0,95 mol pro mol 3-Hydroxy-2-naphthoesäure, eingesetzt.

Natriumcarbonat wird in einer Menge von 0,8 bis 1,8, insbesondere 1,0 bis 1,3 mol pro mol 3-Hydroxy-2-naphthoesäure, eingesetzt.

N-Methylpyrrolidon wird in 3 bis 6, insbesondere 4 bis 5 Volumenteilen je Teil 5-Aminobenzimidazolon, verwendet.

Die Reaktion der Lösung von 3-Hydroxy-2-naphthoesäurechlorid in Xylol bzw. Toluol mit 5-Aminobenzimidazolon wird bei 15 bis 40, insbesondere 20 bis 35°C, durchgeführt.

Die Wasserdampfdestillation wird vorteilhaft mit 5 bis 7 Teilen Wasser und 0,5 bis 0,6 Teilen Natriumcarbonat je Teil 5-Aminobenzimidazolon durchgeführt.

Durch die Wasserdampfdestillation gelingt es überraschenderweise, die Verunreinigungen abzutrennen, so daß der Rückstand nach Absaugen und Waschen mit Wasser sehr reines Zielprodukt darstellt.

Die Dosierzeit des 3-Hydroxy-2-naphthoesäurechlorides ist für die Reinheit des entstehenden Produktes von Bedeutung. Um 3-Hydroxy-N-benzimidazolon-5-yl-naphthoesäure-2-amid in der erforderlichen Pigmentreinheit zu erhalten, sollte die Dosierzeit mindestens 2 h, insbesondere 4 h, bevorzugt zwischen 4 und 10 h betragen. Es ist auch möglich, eine längere Zugabezeit als 10 h zu wählen, jedoch ist damit kein Vorteil bezüglich der Reinheit vorhanden.
Nach dem obengenannten Verfahren kann 3-Hydroxy-N-benzimidazolon-5-yl-naphthoesäure-2-amid in 85 % Ausbeute und in sehr hoher Reinheit, die für die Herstellung von hochechten Azopigmenten erforderlich ist, hergestellt werden. Eine weitere Reinigung, wie nach dem Stand der Technik, kann entfallen.

### Beispiel 1:

### 3-Hydroxy-2-naphthoesäurechlorid in Xylol:

In einem 2l-Vierhalskolben mit Innenthermometer, Gasableitungsrohr und Rührer werden 494,0 g (2,63 mol) 3-Hydroxy-2-napthoesäure und 2470,0 g = 2872 ml Xylol vorgelegt, mit 6,0 g N,N'-Dimethylformamid versetzt, 328,0 g = 201,1 ml (2,76 mol) Thionylchlorid bei 47-50°C über 1 h zudosiert und bis zum Ende der Gasentwicklung 3 h nachgerührt. Man erhält 3181,8 g 3-Hydroxy-2-naphthoesäurechlorid in Xylol als klare Lösung.

### 3-Hydroxy-N-benzimidazolon-5-yl-naphthoesäure-2-amid:

In einem 10 l-Vierhalskolben mit Tropftrichter, Rückflußkühler, Innenthermometer und Rührer werden 1750 ml N-Methylpyrrolidon vorgelegt. Hierzu gibt man 291,5 g (2,75 mol) Natriumcarbonat. Danach werden bei ca. 25°C 372,5 g (2,5 mol) 5-Aminobenzimidazolon zugegeben. In die so erhaltene Suspension werden unter Rühren 3181,8 g 3-Hydroxy-2-naphthoesäurechlorid (aus 494,0 g (2,63 mol) 3-Hydroxy-2-naphthoesäure) in Xylol in 6,5 h bei 25-28°C zudosiert. Nach beendeter Zugabe wird bei Raumtemperatur 30 min nachgerührt. Das ausgefallene Produkt wird abgesaugt (Saugzeit: 50 min) und der Filterkuchen in 10 Portionen mit insgesamt 2150,0 g = 2500 ml Xylol gewaschen.
In einem 6 l-Vierhalskolben mit Rührer, Innenthermometer und Destillationsbrücke mit einem 1 l-Einhalskolben als Vorlage werden 2500 ml Wasser vorgelegt. Danach wird der Filterkuchen eingetragen. Anschließend wird mit 200,0 g Natriumcarbonat versetzt und auf 100°C erhitzt. Es destillieren Xylol und Wasser ab. Das Produkt wird bei 100°C abgesaugt und der Nutschkuchen mehrmals mit insgesamt 6250 ml Wasser neutral gewaschen. Es falllen 1571,4 g Naphtholon wasserfeucht an. Nach dem Trocknen des Filterkuchens bei 100°C/100 Torr erhält man 681,7 g (2,135 mol) 3-Hydroxy-N-benzimidazolon-5-yl-naphthoesäure-2-amid, dies entspricht einer Ausbeute von 85,4 % d. Th. bezogen auf 5-Aminobenzimidazolon.
Das so erhaltene Produkt ist tauglich für die Herstellung von hochechten Azopigmenten und enthält nach HPLC < 0,1 % 5-Aminobenzimidazolon und < 0,1 % 3-Hydroxy-2-naphthoesäure.

### Vergleichsbeispiel 1 (Example aus Dyes and Pigments 12, 57-63 (1990)):

### 3-Hydroxy-2-naphthoesäurechlorid in Chlorbenzol:

99,0 g (0,52 mol) 3-Hydroxy-2-naphthoesäure und 1 ml N,N'-Dimethylformamid werden in 600 ml Chlorbenzol vorgelegt und bei 40°C mit 68,5 g = 42 ml (0,58 mol) Thionylchlorid versetzt. Es wird 1,5 h bei 40°C nachgerührt und anschließend überschüssiges Thionylchlorid unter Vakuum abdestilliert. Es werden 776,8 g 3-Hydroxy-2-naphthoesäurechlorid in Chlorbenzol als klare Lösung erhalten.

### 3-Hydroxy-N-benzimidazolon-5-yl-naphthoesäure-2-amid:

75,0 g (0,5 mol) 5-Aminobenzimidazolon und 41 g wasserfreies Natriumacetat werden in 700 ml N,N'-Dimethylformamid vorgelegt und bei 5°C 776,8 g 3-Hydroxy-2-naphthoesäurechlorid (aus 99,0 g (0,52 mol) 3-Hydroxy-2-naphthoesäure) in Chlorbenzol bei 5-10°C in 30 min zudosiert. Nach beendeter Zugabe wurde 3 h bei 20°C nachgerührt, auf 50°C erwärmt und filtriert. Der Filterkuchen saugt schlecht ab (Saugzeit: 14 h). Zur Reinigung wird der Filterkuchen in 1 l Methanol aufgenommen verrührt, abgesaugt, mit 250 ml Methanol und anschließend mehrmals mit insgesamt 1 l siedendem Wasser gewaschen. Nach dem Trocknen werden 130,3 g 3-Hydroxy-N-benzimidazolon-5-yl-naphthoesäure-2-amid erhalten, dies entspricht 81,7 % d. Th.

Das so erhaltene Produkt ist unbrauchbar für die Herstellung von hochechten Azopigmenten und enthält nach HPLC ca. 0,1 % 5-Aminobenzimidazolon, ca. 0,8 % 3-Hydroxy-2-naphthoesäure und ist zusätzlich verunreinigt.

## Patentansprüche

1. Verfahren zur Herstellung von 3-Hydroxy-N-benzimidazolon-5-yl-naphthoesäure-2-amid, ausgehend von 5-Aminobenzimidazolon und 3-Hydroxy-2-naphthoesäurechlorid, dadurch gekennzeichnet, daß man 5-Aminobenzimidazolon und Natriumcarbonat in N-Methylpyrrolidon vorlegt und eine Lösung von 3-Hydroxy-2-naphthoesäurechlorid in Xylol oder Toluol innerhalb von mindestens 2 Stunden zudosiert, filtriert, mit Xylol oder Toluol wäscht, den Filterkuchen in Wasser, das mit Natriumcarbonat versetzt ist, einträgt und wasserdampfdestilliert, den Rückstand absaugt, wäscht und trocknet.

2. Verfahren nach Anspruch 1, dadurch gekennzeichnet, daß man 5-Aminobenzimidazolon in einer Menge von 0,9 bis 1 mol, bevorzugt 0,95 mol pro mol 3-Hydroxy-2-naphthoesäure einsetzt.

3. Verfahren nach Anspruch 1 oder 2, dadurch gekennzeichnet, daß man Natriumcarbonat in einer Menge von 0,8 bis 1,8, bevorzugt 1,0 bis 1,3 mol pro mol 3-Hydroxy-2-naphthoesäure einsetzt.

4. Verfahren nach mindestens einem der Ansprüche 1 bis 3, dadurch gekennzeichnet, daß man N-Methylpyrrolidon in 3 bis 6, bevorzugt 4 bis 5, Volumenteilen je Teil 5-Aminobenzimidazolon verwendet.

5. Verfahren nach mindestens einem der Ansprüche 1 bis 4, dadurch gekennzeichnet, daß man die Reaktion der Lösung von 3-Hydroxy-2-naphthoesäurechlorid in Xylol bzw. Toluol mit 5-Aminobenzimidazolon bei 15 bis 40, bevorzugt 20 bis 35°C, durchführt.

6. Verfahren nach Anspruch 5, dadurch gekennzeichnet, daß die Dosierzeit mindestens 4 h, bevorzugt 4 bis 10 Stunden, beträgt.

7. Verfahren nach mindestens einem der Ansprüche 1 bis 6, dadurch gekennzeichnet, daß man die Wasserdampfdestillation mit 5 bis 7 Teilen Wasser und 0,5 bis 0,6 Teilen Natriumcarbonat je Teil 5-Aminobenzimidazolon durchführt.

## Claims

1. A process for the preparation of 3-hydroxy-N-benzimidazolon-5-yl-2-naphthamide, starting from 5-aminobenzimidazolone and 3-hydroxy-2-naphthoyl chloride, which comprises initially introducing 5-aminobenzimidazolone and sodium carbonate in N-methylpyrrolidone and metering in a solution of 3-hydroxy-2-naphthoyl chloride in xylene or toluene over the course of at least 2 hours, filtering, washing with xylene or toluene, introducing the filter cake into water to which sodium carbonate has been added and steam-distilling, filtering off the residue with suction, washing and drying.

2. The process as claimed in claim 1, wherein 5-aminobenzimidazolone is employed in an amount of from 0.9 to 1 mol, preferably 0.95 mol, per mole of 3-hydroxy-2-naphthoic acid.

3. The process as claimed in claim 1 or 2, wherein sodium carbonate is employed in an amount of from 0.8 to 1.8, preferably 1.0 to 1.3, mol per mole of 3-hydroxy-2-naphthoic acid.

4. The process as claimed in at least one of claims 1 to 3, wherein N-methylpyrrolidone is used in 3 to 6, preferably 4 to 5, parts by volume per part of 5-aminobenzimidazolone.

5. The process as claimed in at least one of claims 1 to 4, wherein the reaction of the solution of 3-hydroxy-2-naphthoyl chloride in xylene or toluene with 5-aminobenzimidazolone is carried out at 15 to 40°C, preferably 20 to 35°C.

6. The process as claimed in claim 5, wherein the addition time is at least 4 h, preferably 4 to 10 hours.

7. The process as claimed in at least one of claims 1 to 6, wherein the steam distillation is carried out using 5 to 7 parts of water and 0.5 to 0.6 parts of sodium carbonate per part of 5-amino-benzimidazolone.

## Revendications

1. Procédé de préparation de 3-hydroxy-N-benzimidazolon-5-yl-napht-2-amide à partir de la 5-aminobenzimidazolone et du chlorure d'acide 3-hydroxy-2-naphtoïque, caractérisé en ce qu'on place la 5-aminobenzimidazolone et le carbonate de sodium dans de la N-méthylpyrrolidone et on ajoute progressivement, en l'espace d'au moins 2 heures, une solution de chlorure d'acide 3-hydroxy-2-naphtoïque dans du xylène ou du toluène, on filtre, on lave avec du xylène, on introduit le gâteau dans l'eau additionnée de carbonate de sodium, et on entraîne à la vapeur, on essore sous vide le résidu, on lave et on sèche.

2. Procédé selon la revendication 1, caractérisé en ce qu'on utilise la 5-aminobenzimidazolone en une quantité de 0,9 à 1 mole, de préférence de 0,95 mole par mole d'acide 3-hydroxy-2-naphtoïque.

3. Procédé selon la revendication 1 ou 2, caractérisé en ce qu'on utilise le carbonate de sodium en une quantité de 0,8 à 1,8, de préférence de 1,0 à 1,3 mole par mole d'acide 3-hydroxy-2-naphtoïque.

4. Procédé selon au moins l'une des revendications 1 à 3, caractérisé en ce qu'on utilise la N-méthylpyrrolidone en une quantité de 3 à 6, de préférence de 4 à 5, parties volumiques pour chaque partie volumique de 5-aminobenzimidazolone.

5. Procédé selon au moins l'une des revendications 1 à 4, caractérisé en ce qu'on met en oeuvre la réaction de la solution de chlorure d'acide 3-hydroxy-2-naphtoïque dans du xylène ou du toluène avec la 5-aminobenzimidazolone à une température de 15 à 40, de préférence de 20 à 35°C.

6. Procédé selon la revendication 5, caractérisé en ce que la durée d'ajout progressif est d'au moins 4 heures, de préférence de 4 à 10 heures.

7. Procédé selon au moins l'une des revendications 1 à 6, caractérisé en ce qu'on met en oeuvre l'entraînement à la vapeur avec 5 à 7 parties d'eau et 05 à 0,6 parties de carbonate de sodium pour chaque partie de 5-aminobenzimidazolone.
